# EUROPEAN PATENT APPLICATION

(11) **EP 2 452 701 A1**
(43) Date of publication of application: **16.05.2012**
(21) Application number: 10811665.8
(22) Date of filing: 03.08.2010
(51) Int. Cl.: A61L 31/00, A61M 25/00

(54) **MEDICAL DEVICE FOR DELIVERY OF DRUG**

(30) Priority: 27.08.2009 JP 2009196679
(71) Applicant: Terumo Kabushiki Kaisha, Tokyo 151-0072 (JP)
(72) Inventor: NAKAGAWA, Yuuji, Ashigarakami-gun Kanagawa 259-0151 (JP); SHIMOYAMA, Masakazu, Ashigarakami-gun Kanagawa 259-0151 (JP)
(74) Representative: Dossmann, Gérard
(86) International application number: PCT/JP2010/063096
(87) International publication number: WO 2011/024614

(57) **Abstract**

A medical device for safe and sure delivery of drugs to the lesion and a method for production thereof. The medical device has at least part of its surface in contact with the lumen wall tissue of the living body coated with a drug releasing layer containing a drug and a phospholipid, the drug and the phospholipid forming a solid dispersion.

## Description

### TECHNICAL FIELD

Disclosed is a medical device for drug delivery, and a medical device for drug delivery which is so designed as to deliver a drug to a site of treatment for local administration in order to cure the stenosis, occlusion, or arteriosclerotic plaque which has occurred in the lumen of the living body.

### BACKGROUND DISCUSSION

Among treatments for stenosis in the lumen of the living body, such as blood vessel, bile duct, trachea, esophagus, and urethra, Percutaneous Transluminal Angioplasty (PTA) which is intended to expand and keep open the stenosis by means of a balloon catheter or a stent can be conducted. When applied to the stenosis or occlusion in the coronary artery of heart, it is called Percutaneous Transluminal Coronary Angioplasty (PTCA) or Percutaneous Transluminal Coronary Intervention (PCI).

In its early stage, PCI relied upon a balloon catheter only, which method is called Plain Old Balloon Angioplasty (POBA). POBA achieves its object by cracking the blood vessel to expand it. Treatment in this manner can cause coronary artery dissection or recoil which leads to acute coronary occlusion and restenosis, and hence it is unreliable. In fact, restenosis after POBA treatment can occur at a high rate of 40 to 50%. This problem can be addressed by employing a medical device called a stent which is a small hollow tubular object. The indwelling stent in the blood vessel can successfully prevent acute coronary occlusion but can allow restenosis to occur at a rate of 20 to 30%. Thus the problem with restenosis still remained unresolved. This situation has altered with the advent of a Drug Eluting Stent (DES). DES is composed of a stent and a drug (such as immunosuppressive drug and anticancer drug) supported thereon, the drug being gradually and locally released at the stenosis or occlusion in the lumen. Treatment with a DES can remarkably decrease the rate of restenosis to 10% or less.

Unfortunately, DES has posed a new problem with delayed stent thrombosis that can occur more than one year after its placement. This problem has attracted serious attention in the medical world. See, for example, McFadden EP, Lancet, 2004; 364: 1519-1521. A possible reason for the stent thrombosis is that the drug supported on the stent hampers not only the growth of smooth muscle cells but also the formation of nascent intima including endothelial cells. Other factors can include hypersensitivity to the polymer as the carrier of the drug and imperfect stent placement. See, for example, Joner M, J. Am. Coll. Cardiol., 2006; 48: 193-202. In other words, the foregoing problem can arise basically from the fact that the stent, drug, and polymer used for DES remain in the living body for a long period of time, thereby continuously stimulating the blood vessel for a long period of time.

The delayed stent thrombosis can end up with critical situations including frequent cardiac infarction and even death once it occurs. Hence it can be beneficial to provide an adequate countermeasure. A clinical practice to reduce the risk of delayed stent thrombosis is chronic administration of antiplatelet drug, and improved tools for this purpose are appearing as listed below. One employs a biodegradable polymer as the drug carrier to reduce sensitivity against polymer. See, for example, Japanese Patent No. 3,673,973, U.S. Patent No. 5,464,650A, and European Patent Document No. 0 623 354 A1. One controls the drug solubility by elaborating the stent's surface state instead of using a polymer. See, for example, Japanese Patent Document No. JP-T-2004-522559, U.S. Patent Application Publication No. 2002/098278 A1, European Patent Document No. 1 330 273 A1, and International Publication No. WO 03/026718 A1.

On the other hand, an approach is under way to prevent restenosis only by delivering a drug through a balloon catheter without resorting to the indwelling stent or polymer which can cause the delayed stent thrombosis. One way of achieving such an approach is by using a balloon catheter having its balloon surface coated with a composition composed of an anticancer drug (paclitaxel) and a contrast medium (iopromide), the balloon catheter being known as a drug-elution balloon and allowing the drug to migrate to the blood vessel wall of the stenosis part in a short time as the balloon expands. See, for example, Japanese Patent Document No. JP-T-2004-524346, European Patent Document No. 0 499 747 A1, and International Publication No. WO 02/076509 A2; and Japanese Patent Document No. JP-T-2005-538812, U.S. Patent Application Publication No. 2006/020243 A1, European Patent Document No. 1 539 266 A1, and International Publication No. WO 04/028582 A1. In fact, the foregoing balloon catheter has produced good clinical results in treatment for in-stent restenosis. (See, for example, Non-Patent Document 3 and Non-Patent Document 4)

### Prior Art Documents

### Patent Documents

Patent Document 1 : Japanese Patent No. 3,673,973
Patent Document 2: JP-T-2004-522559
Patent Document 3: JP-T-2004-524346
Patent Document 4: JP-T-2005-538812

### Non-Patent Documents

Non-Patent Document 1: McFadden EP, Lancet, 2004; 364:1519-1521
Non-Patent Document 2: Joner M, J. Am. Coll. Cardiol., 2006; 48:193-202
Non-Patent Document 3: Scheller B, New Engl. J. Med., 2006 Nov 16; 355 (20); 2113-2124
Non-Patent Document 4: Tepe G, New Engl. J. Med., 2008 Feb 14; 358 (7): 689-699

### SUMMARY

### TECHNICAL PROBLEM

Certain technologies can have the disadvantage that the contrast medium, which is used in combination with the drug, has a high risk of serious side effects such as dyspnea, sudden pressure reduction, cardiac arrest, and loss of consciousness. In addition, the contrast medium can be soluble in water and hence can be liable to dissolve in blood together with the drug coating the balloon surface, thereby preventing the effective delivery of the drug to the lesion.

According to an exemplary aspect, a medical device is provided for safe and sure delivery of a drug to the lesion.

### TECHNICAL SOLUTION

The present inventors have found that the drug can improve in solubility and effectively migrate into the lumen wall tissue, for example, if it is incorporated with a phospholipid capable of molecular-level dispersion of the drug therein and forming a solid dispersion. After research of the solid dispersion composed of a drug and a phospholipid, the present inventors also found an exemplary solid dispersion compound that is extremely safe and easily wettable albeit insoluble or hardly soluble in water.

Disclosed is a medical device for drug delivery, according to the following exemplary aspects (1) to (11):

(1) A medical device for drug delivery which has at least a part of its surface in contact with the lumen wall tissue of a living body coated with a drug releasing layer containing a drug and a phospholipid, the drug and the phospholipid forming a solid dispersion.

(2) The medical device for drug delivery as defined in paragraph (1) above, wherein the solid dispersion is a solid solution.

(3) The medical device for drug delivery as defined in paragraph (1) or (2) above, wherein the phospholipid is a monomolecular compound.

(4) The medical device for drug delivery as defined in any of paragraphs (1) to (3) above, wherein the phospholipid is at least one species selected from the group consisting of phosphatidylcholine, phosphatidylglycerol, phosphatidic acid, phosphatidylethanolamine, phosphatidylserine, phosphatidylinositol, phosphatidylinositol polyphosphoric acid, sphingomyelin, cardiolipin, partially hydrogenated product thereof, and completely hydrogenated product thereof.

(5) The medical device for drug delivery as defined in any of paragraphs (1) to (4) above, wherein the phospholipid is phosphatidylcholine or a completely hydrogenated product thereof.

(6) The medical device for drug delivery as defined in any of paragraphs (1) to (5) above, wherein the drug is insoluble or hardly soluble in water.

(7) The medical device for drug delivery as defined in any of paragraphs (1) to (6) above, wherein the drug is at least one species selected from the group consisting of anticancer drug, immunosuppressive drug, antibiotic, antirheumatic drug, antithrombotic drug, HMG-CoA reductase inhibitor, insulin resistance improver, ACE inhibitor, calcium antagonist, antihyperlipidemic drug, integrin inhibitor, antiallergic drug, antioxidant, GP IIb/IIIa antagonist, retinoid, flavonoid, carotenoid, lipid improver, DNA synthesis inhibitor, tyrosine kinase inhibitor, antiplatelet drug, antiinflammatory drug, tissue-derived biomaterial, interferon, and nitrogen monoxide generation promoting substance.

(8) The medical device for drug delivery as defined in any of paragraph (1) to (7) above, wherein the drug is paclitaxel.

(9) The medical device for drug delivery as defined in any of paragraphs (1) to (8) above, which is removable from the living body.

(10) The medical device for drug delivery as defined in any of paragraph (1) to (9) above, which is a balloon catheter.

(11) A method for producing the medical device for drug delivery, the method including applying a solution of a drug and a phospholipid dissolved in an organic solvent and subsequently removing the solvent by evaporation, thereby forming a drug releasing layer on at least part of the surface that comes into contact with the lumen wall tissue of the living body.

### ADVANTAGEOUS EFFECT

An exemplary medical device for drug delivery has the drug-releasing layer which contains a drug and a phospholipid, the latter being a low-molecular weight compound capable of molecular-level dispersion of the drug therein, thereby forming a solid dispersion. The solid dispersion can be highly safe and can permit the drug to easily dissolve in and effectively migrate into the lumen wall tissue of the living body.

### BRIEF DESCRIPTION OF DRAWINGS

[Fig. 1] Fig. 1 is a thermogram of differential scanning calorimeter performed on the solid solution obtained in Example 1, according to an exemplary aspect.
[Fig. 2] Fig. 2 is a thermogram of differential scanning calorimeter performed on the mixture obtained in Comparative Example 1, according to an exemplary aspect.
[Fig. 3] Fig. 3 is a thermogram of differential scanning calorimeter performed on the mixture obtained in Comparative Example 2, according to an exemplary aspect.
[Fig. 4] Fig. 4 is a thermogram of differential scanning calorimeter performed on the solid solution obtained in Comparative Example 3, according to an exemplary aspect.
[Fig. 5] Fig. 5 is a thermogram of differential scanning calorimeter performed on paclitaxel (in the form of simple substance) obtained in Comparative Example 4, according to an exemplary aspect.
[Fig. 6] Fig. 6 is a side view of a balloon catheter of a medical device for drug delivery, according to an exemplary aspect.
[Fig. 7] Fig. 7 is a partly cut-away external view of the balloon catheter shown in Fig. 6, with the proximal shaft 8 partly omitted and the major constituents enlarged, according to an exemplary aspect.
[Fig. 8] Fig. 8 is an enlarged sectional view of the balloon taken along the line B-B in Fig. 7, according to an exemplary aspect.
[Fig. 9] Fig. 9 is a thermogram of differential scanning calorimeter performed on the solid solution obtained in Example 13, according to an exemplary aspect.
[Fig. 10] Fig. 10 is a thermogram of differential scanning calorimeter performed on the solid solution obtained in Example 14, according to an exemplary aspect.
[Fig. 11] Fig. 11 is a thermogram of differential scanning calorimeter performed on the solid solution obtained in Comparative Example 12, according to an exemplary aspect.
[Fig. 12] Fig. 12 is a thermogram of differential scanning calorimeter performed on the solid solution obtained in Comparative Examples 13, according to an exemplary aspect.

### DETAILED DESCRIPTION

According to an exemplary aspect, a medical device for drug delivery is provided which has at least a part of its surface in contact with the lumen wall tissue of a living body coated with a drug releasing layer containing a drug and a phospholipid, the drug and the phospholipid forming a solid dispersion.

The phospholipid can have adequately controlled water solubility and wettability owing to, for example, its well-balanced hydrophilicity and lipophilicity to be bonded for the phosphate ester moiety and the fatty acid moiety. It is exemplified by hydrogenated soybean lecithin, which is commonly available. For example, this lecithin is hardly soluble in water but wettable when wet, so that the phospholipid forms, in combination with a drug, a hardly water-soluble solid dispersion which can prevent the drug from dissolving in blood when the medical device for drug delivery approaches the lesion percutaneously and intravenously. The solid dispersion can become wet with water in blood, thereby increasing in viscosity, so that it helps the medical device for drug delivery to adhere to the surface of the lumen wall tissue when it comes into contact with the lumen wall tissue at the lesion. The solid dispersion can adhere to the surface of the lumen wall tissue and stay there without being carried off by blood and releases the drug into the tissue. The phospholipid, for example, that of monomolecular compound, can be easily soluble in the cell membrane so that it permits the drug to migrate efficiently into cells.

In addition, the above-mentioned phospholipid can be an extremely safe compound in view of the fact that it exists in the living body as a constituent of the cell membrane and it is an approved pharmaceutical additive and food additive (as in the case of soybean lecithin) and it can be made into liposomes for clinical use.

In an exemplary embodiment where the medical device for drug delivery is a hollow expandable body, the solid dispersion composed of a drug and a phospholipid can exist entirely or partly on the surface of the hollow expandable body. As the hollow expandable body comes into contact with the arteriosclerotic plaque in the lumen, the drug and the phospholipid can migrate into the cells of the lumen wall tissue.

In an exemplary embodiment where the medical device for drug delivery is a balloon catheter, the solid dispersion composed of a drug and a phospholipid can exist entirely or partly on the surface of the balloon. As the balloon catheter is expanded on the stenosis or occlusion in the lumen, the solid dispersion composed of the drug and the phospholipid, can migrate into the cells of the lumen wall tissue.

A detailed description is given below of an exemplary drug and an exemplary phospholipid which constitute an exemplary solid dispersion.

### (Drug)

The drug may be selected without specific restrictions so long as, for example, it treats or cures the stenosis, occlusion, or arteriosclerotic plaque which has occurred in the lumen of the living body. For example, the drug can be hardly soluble or insoluble in water from the standpoint of its limited elution into blood.

The drug can be at least one species selected from the group including anticancer drug, immunosuppressive drug, antibiotic, antirheumatic drug, antithrombotic drug, HMG-CoA reductase inhibitor, insulin resistance improver, ACE inhibitor, calcium antagonist, antihyperlipidemic drug, integrin inhibitor, antiallergic drug, antioxidant, GP IIb/IIIa antagonist, retinoid, flavonoid, carotenoid, lipid improver, DNA synthesis inhibitor, tyrosine kinase inhibitor, antiplatelet drug, antiinflammatory drug, tissue-derived biomaterial, interferon, and nitrogen monoxide generation promoting substance. The drugs listed above can control the behavior of the cells of the lesion tissue and treat or cure the lesion.

The anticancer drug can include, for example, vincristine, vinblastine, vindesine, irinotecan, pirarubicin, paclitaxel, docetaxel, methotrexate and the like,

The immunosuppressive drug can include, for example, sirolimus and derivatives thereof, such as everolimus, pimecrolimus, ABT-578, AP23573, and CCI-779; tacrolimus, azathioprine, cyclosporine, cyclophosphamide, mycophenolate mofetil, gusperimus, mizoribine and the like.

The antibiotic can include, for example, mitomycin, adriamycin, doxorubicin, actinomycin, daunorubicin, idarubicin, pirarubicin, aclarubicin, epirubicin, peplomycin, zinostatin stimalamer and the like.

The antirheumatic drug can include, for example, methotrexate, sodium thiomalate, penicillamine, lobenzarit and the like.

The antithrombotic drug can include, for example, heparin, aspirin, antithrombin drug, ticlopidine, hirudin and the like.

The HMG-CoA reductase inhibitor can include, for example, cerivastatin, cerivastatin sodium, atorvastatin, atorvastatin calcium, rosuvastatin, rosuvastatin calcium, pitavastatin, pitavastatin calcium, fluvastatin, fluvastatin sodium, simvastatin, lovastatin, pravastatin, pravastatin sodium and the like.

The insulin resistance improver can include, for example, thiazolidine derivatives, such as troglitazone, rosiglitazone, pioglitazone and the like.

The ACE inhibitor can include, for example, quinapril, perindopril erbumine, trandolapril, cilazapril, temocapril, delapril, enalapril maleate, lisinopril, captopril and the like.

The calcium antagonist, can include, for example, nifedipine, nilvadipine, diltiazem, benidipine, nisoldipine and the like.

The antihyperlipidemic drug can include, for example, bezafibrate, fenofibrate, ezetimibe, torcetrapib, pactimibe, K-604, implitapide, probucol and the like.

The integrin inhibitor can include, for example, AJM300.

The antiallergic drug can include, for example, tranilast and the like.

The antioxidant can include, for example, a-tocopherol, catechin, dibutylhydroxytoluene, butylhydroxyanisol and the like.

The GP IIb/IIIa antagonist can include, for example, abciximab and the like.

The retinoid can include, for Example, all-trans retinoic acid and the like.

The flavonoid can include, for example, epigallocatechin, anthocyanin, proanthocyanidin and the like.

The carotenoid can include, for example, β-carotene lycopene and the like.

The lipid improver can include, for example, eicosapentaenoic acid.

The DNA synthesis inhibitor can include, for example, 5-FU.

The tyrosine kinase inhibitor can include, for example, genistein, tyrphostin, erbstatin,staurosporine and the like.

The anti platelet drug can include, for example, ticlopidine, cilostazol, clopidogrel and the like.

The drug can include, for example, a steroid, dexamethasone, prednisolone and the like.

The tissue-derived biomaterial can include, for example, EGF (epidermal growth factor), VEGF (vascular endothelial growth factor), HGF (hepatocyte growth factor), PDGF (platelet derived growth factor), BFGF (basic fibroblast growth factor) and the like.

The interferon can include, for example, interferon-yl a.

The nitrogen monoxide promoting substance can include, for example, L-arginine.

In an exemplary embodiment the drug can include a drug that can be used to treat or cure stenosis and that can rapidly migrate into cells such as, for example, paclitaxel, docetaxel, sirolimus, everolimus, atorvastatin, and simvastatin, for example, paclitaxel.

### (Phospholipid)

The phospholipid is not necessarily specifically restricted. The phospholipid can be a monomolecular compound from the standpoint of an ability to combine with the drug to form the solid dispersion and an ability to cause the drug to rapidly migrate into cells. For example, the phospholipid can be at least one species selected from the group including phosphatidylcholine, phosphatidylglycerol, phosphatidic acid, phosphatidylethanolamine, phosphatidylserine, phosphatidylinositol, phosphatidylinositol polyphosphoric acid, sphingomyelin, cardiolipin, partially hydrogenated product thereof, and completely hydrogenated product thereof.

For example, the phospholipid can include at least one of phosphatidylcholine and completely hydrogenated product of phosphatidylcholine.

The phosphatidylcholine mentioned above can be one which has a C8-20 aliphatic acyl group so that it is hardly soluble in water but is wettable. The aliphatic acyl group can be exemplified by octanoyl group, azelaoyl group, decanoyl group, lauroyl group, myristoyl group, palmitoyl group, stearoyl group, and oleoyl group.

The ratio of the phospholipid to the drug can be 10 to 200 mass%. For example, when the ratio of the phospholipid is lower than 10 mass%, the phospholipid and the drug will not form the solid dispersion. For example, when the ratio of the phospholipid higher than 200 mass%, the resulting drug releasing layer is thick due to excessive phospholipid, which can be detrimental to smooth insertion into the body and smooth passage through the lesion.

An exemplary ratio of the phospholipid to the drug is 30 to 180 mass%, for example, 50 to 150 masts%, so that they form a solid solution which is an exemplary form of the solid dispersion. The solid solution is defined as a form of solid dispersion in which one component is entirely dispersed in the other component at the molecular level as if the resulting system is chemically and physically uniform as a whole. For example, when the drug and the phospholipid for the solid solution, a beneficial effect can be produced.

An exemplary solid dispersion composed of the drug and the phospholipid may be examined for its structure by powder X-ray diffractometry (PXRD), differential scanning calorimetry (DSC), or solid nuclear magnetic resonance (NMR). For example, in the case where the solid solution is formed, the melting peak of the drug can disappear in DSC, for example, a thermogram of DSC performed on the solid dispersion has no endothermic peak due to the fusion of the drug.

The solid dispersion can be prepared by any suitable method. An exemplary method includes dissolving the drug and the phospholipid in an organic solvent such as methanol, ethanol, chloroform, tetrahydrofuran, hexane, cyclohexane and the like, to give a solution containing 0.001 to 20 mass%, for example, 0.1 to 10 mass%, of the two components in total, and removing the organic solvent by drying in vacuum or with heating.

The solid dispersion of the drug and the phospholipid may be a porous body so that the phospholipid is easily wettable. The porous body may be prepared in any suitable manner. An exemplary method includes applying the solution containing the drug and the phospholipid to the medical device and removing the solvent used for the solution by freeze-drying with liquid nitrogen and the like.

### Drug releasing layer

The drug releasing layer can contain the solid dispersion composed of the drug and the phospholipid. It can also optionally contain pharmaceutical additives such as, for example, a stabilizer, antioxidant, filler and the like. The drug releasing layer can partially or entirely cover the surface of the member constituting the medical device. In an exemplary embodiment, it can be sufficient for the drug releasing layer to partly cover the surface of the member constituting the medical device.

The drug releasing layer can have an adequate thickness which can be selected from the standpoint of ease with which the medical device approaches (is delivered to) the lesion, wherein the performance of the medical device can include stimulus to the blood vessel wall, and the effect of the released drug. The drug releasing layer can have a thickness of 0.1 to 200 µm, for example, 1 to 100 µm, for example, 10 to 50 µm The thickness in this range can be suitable for the medical device for drug delivery to effectively release the drug when it comes into contact with the lumen of the living body. With such a thickness, the medical device for drug delivery can, for example, have a reduced outside diameter, and the medical device for drug delivery can smoothly approach the lesion and prevent restenosis without stimuli to the blood vessel wall.

The drug releasing layer may be formed in any suitable manner. An exemplary method includes applying the solution containing the drug and the phospholipid to the surface of the medical device by means of microsyringe, microdispenser, ink jet, or spray, and subsequently removing the organic solvent used for the solution by drying in vacuum or with heating. In this way, for example, it is possible to form the solid dispersion composed of the drug and the phospholipid which functions as the drug releasing layer that covers at least partly the surface of the medical device in contact with the lumen tissue of the living body thereby manufacturing an exemplary medical device for drug delivery.

In an exemplary method of forming the drug releasing layer, the surface of the base material such as balloon can be coated with an adhesive in advance and then the solution containing the drug and the phospholipid can be applied to the adhesive layer. This procedure can be carried out in order to ensure good adhesion between the drug releasing layer which contains the solid dispersion composed of the drug and the phospholipid, and the surface of the balloon. The coating material may contain a polymer having phospholipid on its side chains. Such a polymer can function as a good binder excelling in adhesion to the balloon and compatibility with the solid dispersion composed of the drug and the phospholipid.

### Medical device for drug delivery

The medical device for drug delivery can include any suitable structure. For example, it can include a hollow expandable body that expands after it has been inserted into the desired position. For example, it can be a balloon catheter, stent, snare-shaped wire, thrombus-removing basket or the like.

The following is a detailed description of an exemplary walloon catheter as an exemplary medical device for drug delivery.

### (Balloon catheter)

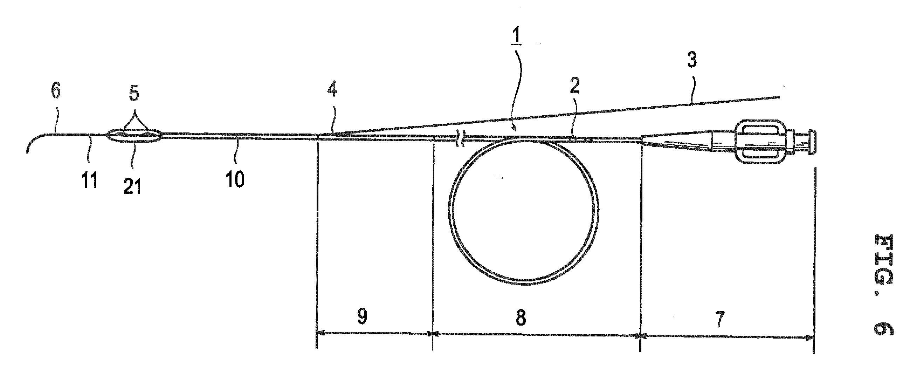

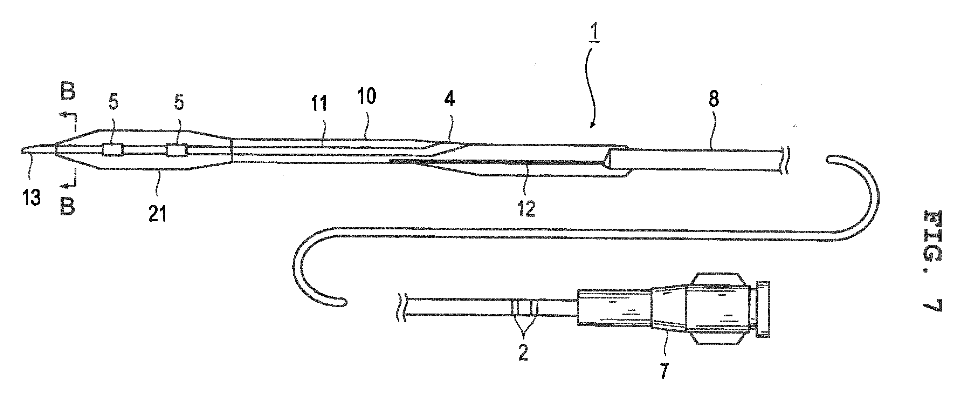

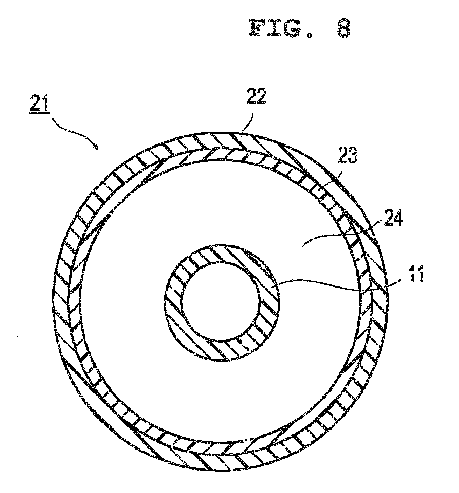

Fig. 6 is a side view of an exemplary balloon catheter as one example of a medical device for drug delivery. Fig. 7 is a partly cut-away external view of the balloon catheter shown in Fig. 6, with the proximal shaft 8 partly omitted and exemplary constituents enlarged. Fig. 8 is an enlarged sectional view of the balloon taken along the line B-B in Fig. 7. The balloon catheter can be classified into two large categories: rapid exchange type and over-the-wire type. Figs. 6 and 7 show the balloon catheter of rapid exchange type as an example. Exemplary aspects can be applied to balloon catheters of any suitable type. The following is a detailed description of exemplary constituents of an exemplary balloon catheter.

The exemplary balloon catheter 1 shown in Figs. 6 and 7 is that of a rapid exchange type. It is so designed as to be inserted into the blood vessel along the guide wire 3 or 6. The balloon catheter 1 is composed of the hub 7, the proximal shaft 8, the intermediate part 9, the distal shaft 10, the balloon 21, and the inner shaft 11, which are sequentially arranged from the base end.

The hub 7 adjacent to the base end has the lure taper for connection with a compressing apparatus such as inflator. The hub 7 is joined to the proximal shaft 8 which can be made of a comparatively stiff material such as metal or special plastics in such a way that a fluid can pass through the joint. The proximal shaft 8 can be provided with the depth marker 2 that permits the operator to know easily how far the balloon catheter 1 has been inserted into the guiding catheter (not shown) during angioplasty. The fore-end of the proximal shaft 8 is the reinforcing member 12. Between the reinforcing member 12 and the unworked part is an inflation opening that permits the fluid to flow across the wall of the proximal shaft 8. Thus, the fluid forced in from the hub 7 flows from the inside to the outside of the proximal shaft 8 through the inflation opening. (The outside is in the lumen at the intermediate part 9.) The inflation opening may be omitted, in which case the fluid flows in the reinforcing member from the base end to the fore-end of the reinforcing member and flows into the outside (which is in the lumen at the distal shaft 10).

The fore-end of the proximal shaft 8 is connected to the intermediate part 9 in such a way that a fluid can pass through the connecting point. The fore-end of the intermediate part 9 can be connected to the distal shaft 10 with comparatively low stiffness which is made of plastics in such a way that a fluid can pass through the connecting point. The fore-end of the distal shaft 10 is connected to the base end of the balloon 21 in such a way that a fluid can pass through the connecting point.

The distal shaft 10 and the balloon 21 have the inner shaft 11 which passes through them coaxially. The inner shaft 11 has the distal tip 13 at its fore-end. The distal tip 13 extends from the fore-end of the balloon 21 and fluid-tightly joins with the fore-end of the balloon 21. The base end of the inner shaft 11 extends to and fluid-tightly joins with the guide wire opening 4 which is formed at one part between the intermediate part 9 and the distal shaft 10. The guide wire 3 or 6 in Fig. 6 passes the inner shaft 11 from the fore-end opening of the distal tip 13 to the guide wire opening 4. The inner shaft 11 inside the balloon 21 can have contrast markers 5 on its periphery.

The balloon 21 remains collapsed around the inner shaft 11 when it is not yet expanded. Alter expansion, the balloon 21 can take on a cylindrical shape at its center so that it easily expands the stenosed part of the blood vessel. The central part of the balloon 21 does not always need to be completely cylindrical but it may be polygonal columnar. The contrast markers 5 can permit the operator to easily locate the balloon 21 at the stenos part by X-ray radioscopy for angioplasty.

The exemplary balloon catheter shown in Figs. 6 and 7 is that of double-tube structure including the distal shaft 10 and the inner shaft 11, However, it may be constructed such that the inner shaft is replaced by a solid rod-like supporting member without being restricted the foregoing structure. An exemplary balloon can be formed from a cylindrical balloon film 23 having a thickness of about 5 to 50 µm. The balloon expands and shrinks as it is supplied with and emptied of the compressed fluid that passes through the lumen 24, which is a space existing between the outside of the inner shaft and the inside of the distal shaft, as shown in Fig. 8. The exemplary balloon of the exemplary balloon catheter has a hollow sectional structure as shown in Fig. 8. It has the balloon film 23 coaxially surrounding the inner shaft 11, and the balloon film 23 is coated with the drug releasing layer 22 which contains the solid dispersion composed of the drug and the phospholipid.

An exemplary balloon film constituting the balloon can be formed from a slightly material so that it can expand the stenosed part of the blood vessel. Examples of such a material include polyolefin (such as polyethylene, polypropylene, polybutene, ethylene-propylene copolymer, ethylene-vinyl acetate copolymer, ionomer and the like), crosslinked polyolefin, polyester (such as polyethylene terephthalate and the like), polyester elastomer, polyvinyl chlorine, polyurethane, polyurethane elastomer, polyphenylenesulfide, polyamide, polyamide elastomer, polymeric material such as fluoroplastics and the like, silicone rubber, latex robber and the like. These polymeric materials may be used in the form of laminate film. The balloon 21 may be formed separately to be attached to the tip of the distal shaft 10 by biaxially-oriented blow moulding, or it may be formed integral with the tip of the distal shaft 10 by stretch blow molding.

The balloon 21 can be formed such that its cylindrical part in its expanded state has an outside diameter of, for example, 1.0 to 10 mm, for example, 1.0 to 5.0 mm, and a length of 5 to 50 mm, for Example, 10 to 40 mm, with the overall length being, for example, 10 to 70 mm, for example, 15 to 60 mm.

The balloon catheter may be produced by any suitable method. Exemplary methods include a dripping method including dripping a mold in a solution of raw material, whereby forming the balloon film on the mo!d, followed by drying and denoting, and a blow moulding method including blowing a parison, thereby forming the balloon film.

### Examples

The effect of the invention will be described in more detail with reference to the following examples and comparative Examples, which are not intended to restrict the scope thereof.

### (Example 1 and Comparative Examples 1 to 4)

For the purpose of screening adequate solid dispersions, several exemplary mixtures were prepared, each composed of paclitaxel and a compound of different kind, and these mixtures underwent differential scanning calorimetry (DSC) using a differential scanning calorimeter.

In Example and Comparative Examples 1 to 3, each mixture with the composition shown in Table 1 was dissolved in 0.5 mL of ethanol or acetone. In Comparative Example 4, 10 mg of paclitaxel alone was dissolved in 0.5 mL of acetone. The paclitaxel is a product of SIGMA Corp.

The thus prepared solution was completely freed of ethanol or acetone by vacuum drying. The resulting dry product was placed in an aluminum pan and thereafter underwent DSC in a nitrogen atmosphere at a heating rate of 10°C/min. The apparatus used for DSC is Diamond DSC made by Perkin Elmer Inc. The results are shown in Table 1.

**Table 1**

| | Compound * | Amount of compound | Amount of paclitaxel | Solvent for solution | Peak due to fusion of paclitaxel |
|---|---|---|---|---|---|
| Example 1 | HSPC | 10 mg | 10 mg | Ethanol | No |
| Comparative Example 1 | PVP | 10 mg | 10 mg | Acetone | Yes |
| Comparative Example 2 | BHT | 10 mg | 10 mg | Acetone | Yes |
| Comparative Example 3 | BHA | 10 mg | 10 mg | Acetone | No |
| Comparative Example 4 | None | --- | 10 mg | Acetone | Yes |

| | | | | | |
|---|---|---|---|---|---|
| * HSPC: Hydrogenated soybean phospholipid (NOF Corporation) PVP: Polyvinyl pyrrolidone (BASF SE) BHT: Dibutylhydroxytoluene (Wako Pure Chemical Industries, Ltd.) BHA: Butylhydroxyanisole (Wako Pure Chemical Industries, Ltd.) | | | | | |

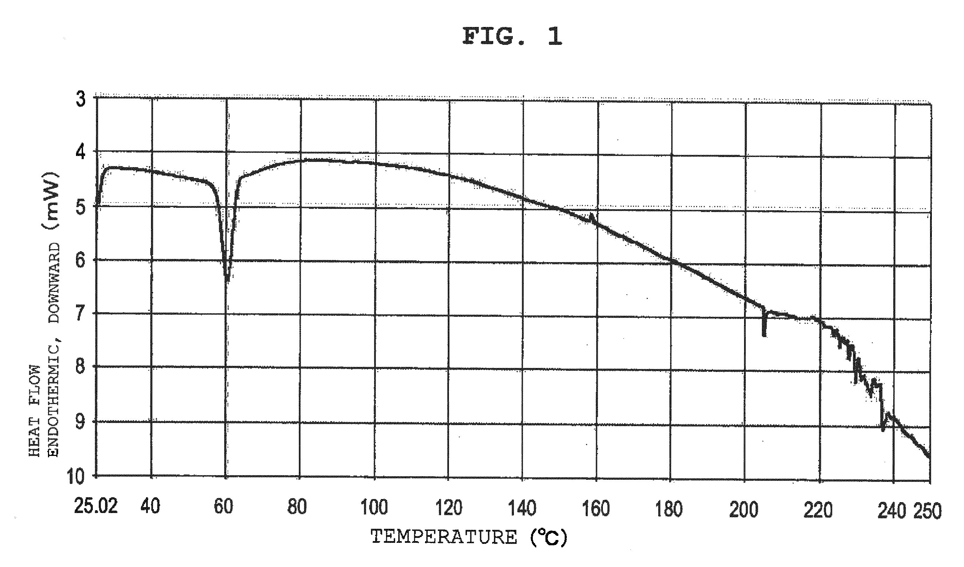

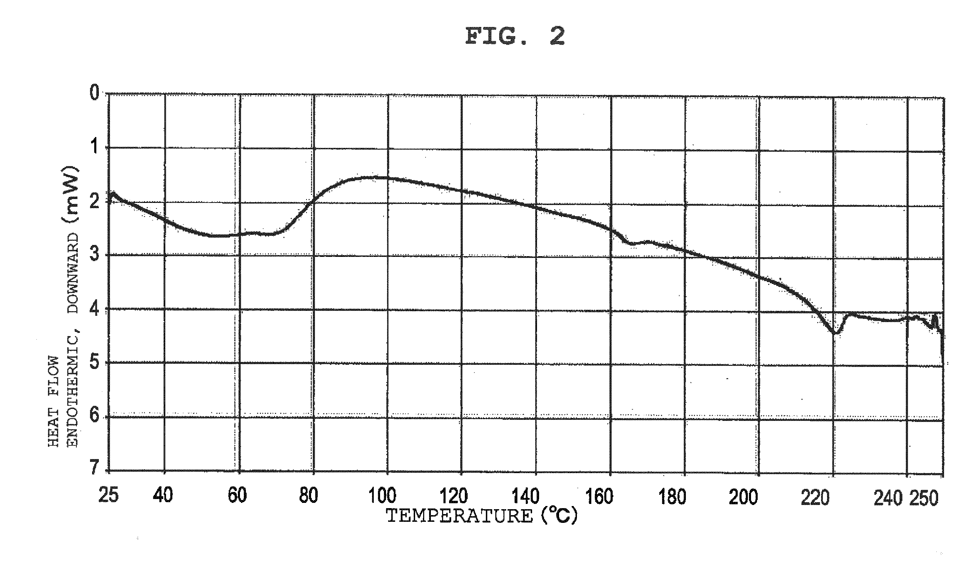

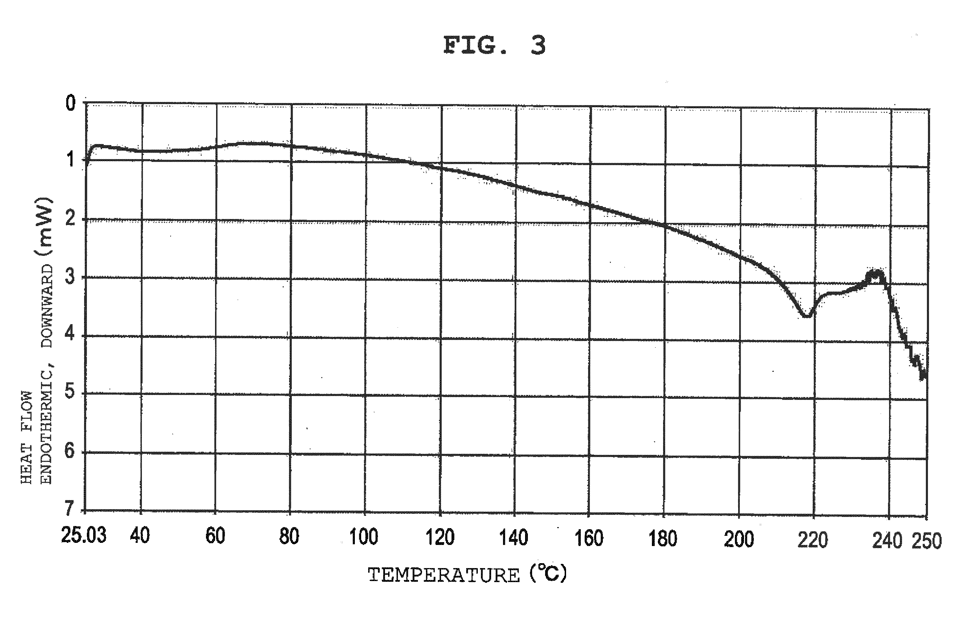

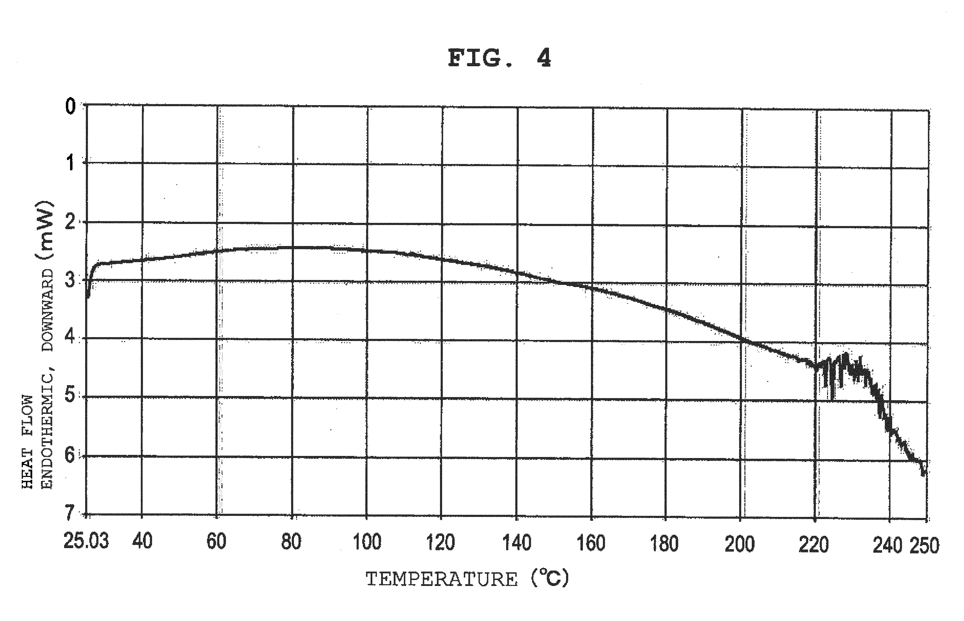

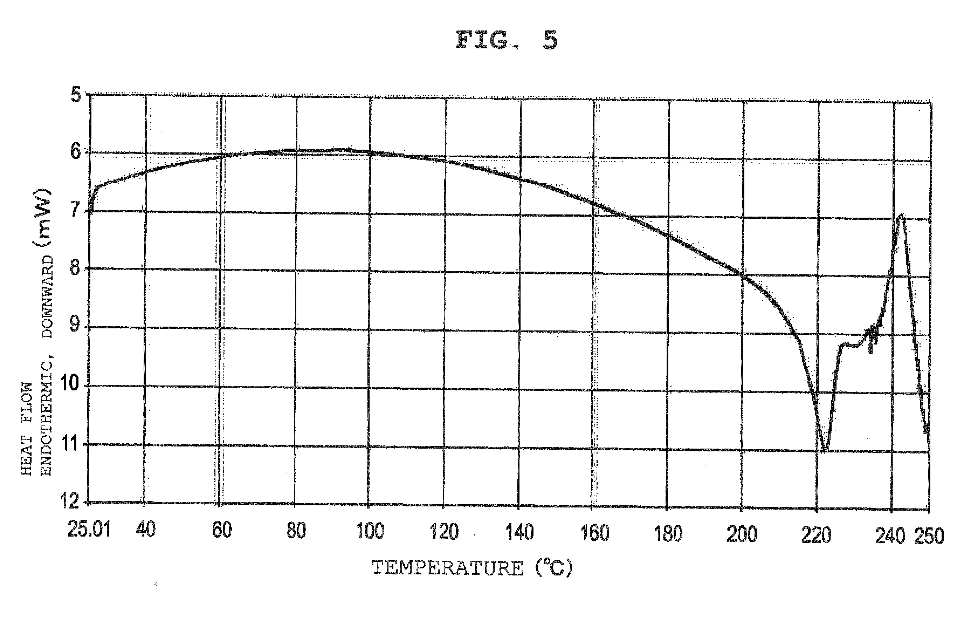

It is apparent from Table 1 that the mixture gave an endothermic peak at about 220°C due to fusion of paclitaxel in Comparative Examples 1, 2, and 4, whereas the mixture gave no endothermic peak in Example 1 and Comparative Example 3. Figs. 1 to 5 show respectively the DSC thermograms of the solid solutions and mixtures obtained in Example 1 and Comparative Examples 1 so 4.

The foregoing results suggest that hydrogenated soybean phospholipid (HSPC) and butylhydroxyanisole (BHA) are completely miscible with paclitaxel to form a solid solution.

### (Examples 2 to 6 and Comparative Example 5 to 9)

For the purpose of observing the relationship between the amount of HSPC or BHA and the properties of the solid solution of paclitaxel, there were prepared mixtures of HSPC and paclitaxel mixtures of BHA and paclitaxel in various ratios as shown in Table 2, and the resulting mixtures were examined by DSC.

Each sample in Examples 2 and 3 and Comparative Example 5 gave an endothermic peak due to fusion of paclitaxel whereas each sample in Examples 4 to 6 and Comparative Examples 6 to 9 gave no endothermic peak. This suggests that HSPC more than 30 mass% or BHA more than 20 masts% forms a solid solution with paclitaxel.

**Table 2**

| | Amount of HSPC | Amount of BHA | Amount of paclitaxel | Peak due to fusion or paclitaxel |
|---|---|---|---|---|
| Example 2 | 1 mg | --- | 10 mg | Yes |
| Example 3 | 2 mg | --- | 10 mg | Yes |
| Example 4 | 3 mg | --- | 10 mg | No |
| Example 5 | 5 mg | --- | 10 mg | No |
| Example 6 | 10 mg | --- | 10 mg | No |
| Comparative Example 5 | --- | 1 mg | 10 mg | Yes |
| Comparative Example 6 | --- | 2 mg | 10 mg | No |
| Comparative Example 7 | --- | 3 mg | 10 mg | No |
| Comparative Example 8 | --- | 5 mg | 10 mg | No |
| Comparative Example 9 | --- | 10 mg | 10 mg | No |

### (Examples 7 to 11)

For the purpose of observing the relationship between the molecular structure of the phospholipid and the properties of the solid solution of paclitaxel, there were prepared mixtures of paclitaxel and phosphatidylcholine (PC) (made by NOF Corporation) differing in the aliphatic acyl group as shown in Table 3, and the resulting mixtures were examined by DSC in the same way as in Example 6.

The results show that all the samples in Examples 7 to 11 gave no endothermic peak due to fusion of paclitaxel. This suggests that PC forms a solid solution with paclitaxel regardless of the type of its aliphatic acyl group (varying in carbon number, with or without unsaturated bond).

**Table 3**

| | Aliphatic acyl group of PC | Amount of PC | Amount of paclitaxel | Peak due to fusion |
|---|---|---|---|---|
| Example 7 | lauroyl group | 10 mg | 10mg | No |
| Example 8 | Myristoyl group | 10mg | 10mg | No |
| Example 9 | Palmitoyl roup | 10 mg | 10 mg | No |
| Example 10 | Stearoyl group | 10 mg | 10 mg | No |
| Example 11 | Oleoyl group | 10 mg | 10 mg | No |

### (Example 12 and Comparative Examples 10 and 11)

For the purpose of observing the relationship between the properties of the solid solution of paclitaxel and the ability of paclitaxel to migrate into the tissue, there were prepared a composition of HSPC and paclitaxel and a composition of BHA and paclitaxel, and the resulting compositions were applied to a balloon catheter. The coated balloon catheter was inserted into the rabbit iliac artery and expanded there, and the blood vessel tissue was examined for the content of paclitaxel.

### (a) Coating onto the balloon catheter

In Example 12 and Comparative Example 10, each of the solution of HSPC and paclitaxel and the solution of BHA and paclitaxel, which were prepared in Example 1 and Comparative Example 3, respectively, was applied to the external surface of the balloon member of the balloon catheter (made by Terumo Corporation). The balloon member is cylindrical in shape, measuring 3.0 mm in outside diameter and 20 mm in length, and is made of polyamide. Coating was accomplished by using a microsyringe pump (mad by KD Scientific), with the balloon catheter rotating. This coating step was followed by vacuum drying to completely remove ethanol or acetone thereby forming a coating layer. The thus coated balloon was enclosed in a stent (made by Terumo Corporation), which was subsequently crimped. The balloon catheter havin the crimped stent was sterilized with ethylene oxide gas. In Comparative Example 11, the same procedure as above was repeated except that the solution was replaced by the solution of paclitaxel alone which was prepared in Comparative Example 1.

The sterilized balloon catheter was examined for the amount of paclitaxel carried thereon by means of high-performance liquid chromatography (HPLC, made by Shimadzu Corporation). The results are as follows.
529 µg in Examples 12 which employed the solution of HSPC and paclitaxel.
514 µg in Comparative Examples 10 which the solution of BHA and paclitaxel.
548 µg in Comparative Example 11 which employed the solution of paclitaxel alone.

(b) Expansion of the coated balloon in rabbit iliac artery The balloon catheter with coating which was prepared as mentioned above was inserted into the rabbit iliac artery with the help of a guiding catheter (5 Fr) and a guide wire for PTCA (both made by Terumo Corporation). The balloon was expanded so that its outside diameter reached 3.0 mm and was left expanded for 60 seconds. The stent, which had been crimped on the balloon, was left in the iliac artery. After blood flow for 60 minutes, the blood vessel with the indwelling stent was extracted.

(c) Determination of paclitaxel in the extracted blood vessel The extracted blood vessel with the indwelling stent was treated with acetonitrile for extraction of paclitaxel. The amount of paclitaxel was determined by HPLC. The results are shown in Table 4 below.

**stable 4**

| | Solid solution | Content of paclitaxel in blood vessel tissue |
|---|---|---|
| Example 12 | HSPC/paciitaxel | 140 µg |
| Comparative Example 10 | BHA/paclitaxel | 64 µg |
| Comparative Example 11 | Paclitaxel alone | 64 µg |

It is apparent from Table 4 that the solid solution of HSPC and paclitaxel permits paclitaxel to migrate to the blood vessel tissue much more efficiently than the solution of paclitaxel alone. By contrast, the solid solution of BHA and paclitaxel does not differ from the solution of paclitaxel alone. While not wishing to be bound by any particular theory, a possible reason for this is that the solid solutions differ in water wettability. That is, the solid solution of BHA and paclitaxel is hardly wettable and hence unable to adhere to the blood vessel tissue, whereas the solid solution of HSPC and paclitaxel easily becomes wet upon contact with water and adheres to the blood vessel tissue and stays on the surface of the blood vessel. As a result, the solid solution of HSPC and paclitaxel is assumed to be liable to migrate to the tissue.

### (Examples 13 and 14 and Comparative Examples 12 and 13)

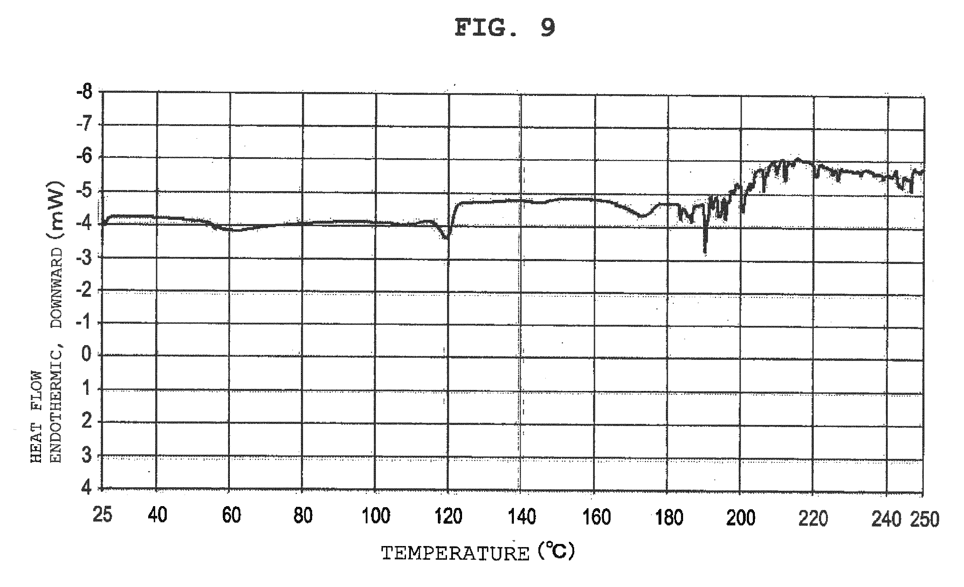

For the purpose of observing the properties of the solid solution of HSPC and a drug other than paclitaxel, there were prepared a mixture of HSPC and sirolimus and a mixture of HSPC and simvastatin, as shown in Table 5 below. These mixtures underwent DSC in the same way as in Example 1. Sirolimus and simvastatin in the form of simple substance (without HSPC) also underwent DSC in the same way as in Comparative Example 4. Examples 13 and 14 and Comparative Examples 12 and 13 gave DSC thermograms as shown in Figs. 9 to 12.

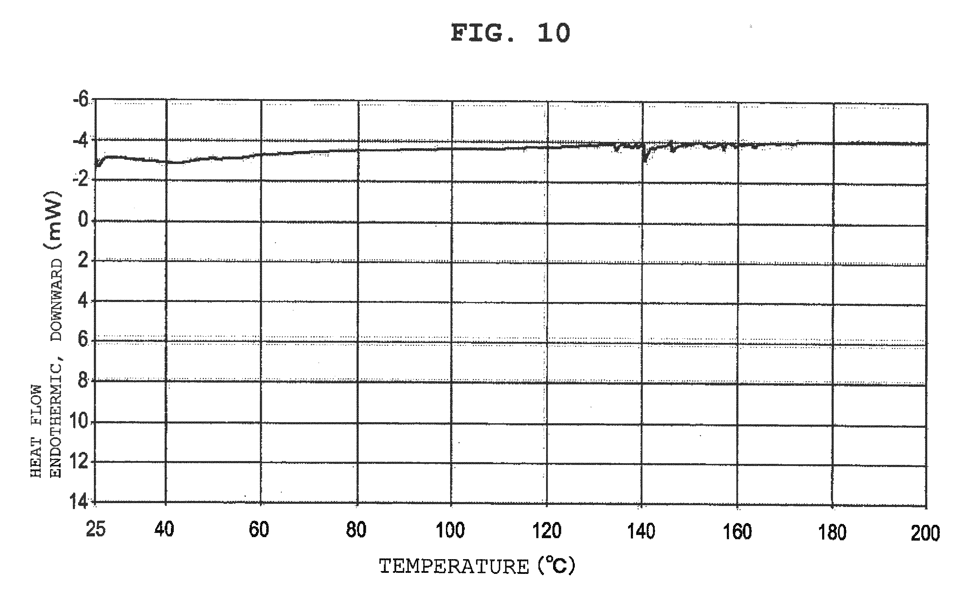

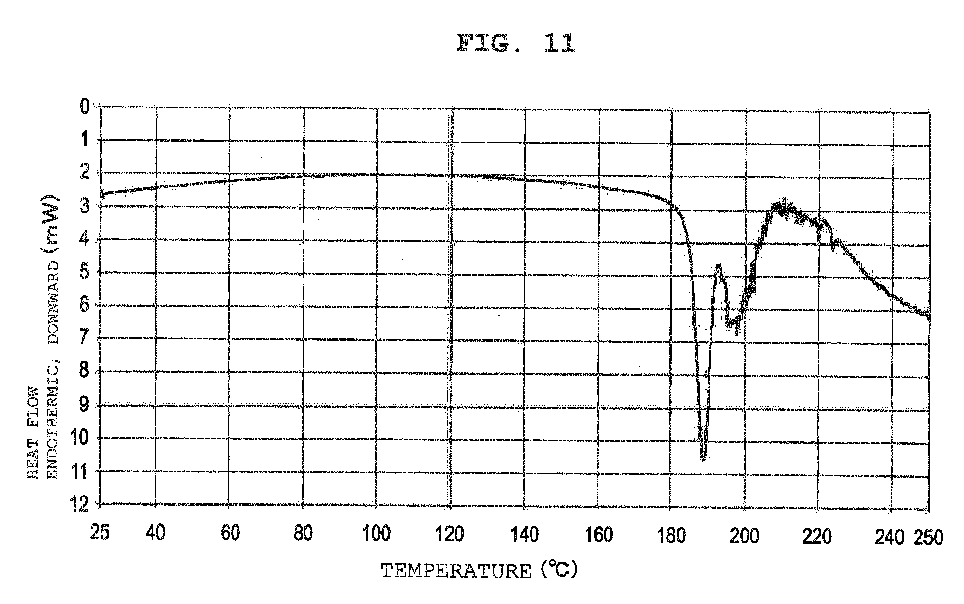

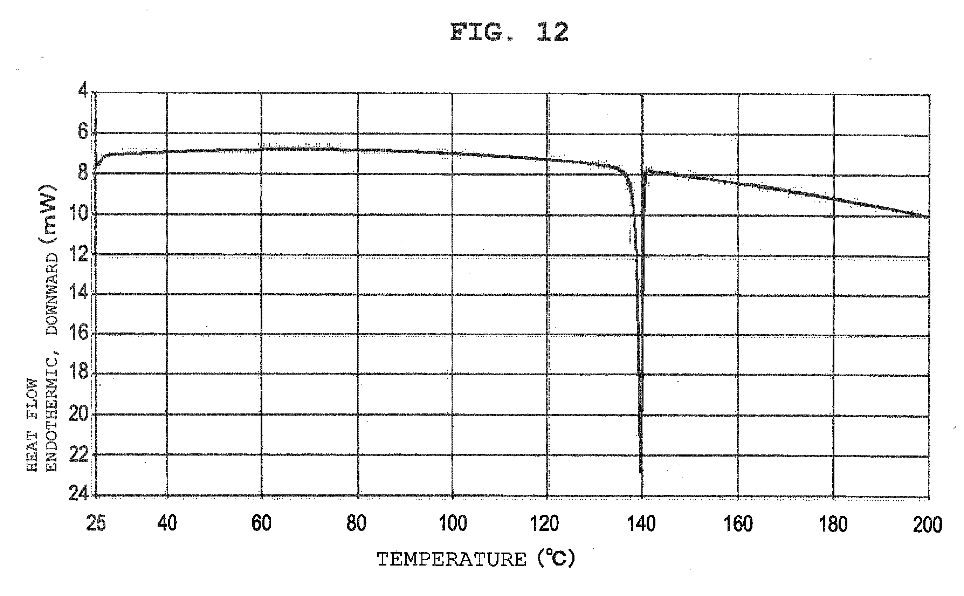

Fig. 11 shows that the thermogram for Comparative Example 12 has an endothermic peak at about 185°C due to fusion of sirolimus, whereas Fig. 9 shows that the thermogram for Example 13 does not have an endothermic peak due to fusion of sirolimus. Likewise, Fig. 12 shows that the thermogram for Comparative Example 13 has an endothermic peak at about 140°C due to fusion of simvastatin, whereas Fig. 10 shows that the thermogram for Example 14 does not have an endothermic peak due to fusion of simvastatin.

The foregoing results suggest that HSPC is highly compatible with not only paclitaxel but also sirolimus and simvastatin and forms a solid solution with them.

**Table 5**

| | Compound | Amount of compound | Drug * | Amount of drug | Peak due to fusion of drug |
|---|---|---|---|---|---|
| Example 13 | HSPC | 10 mg | Sirolimus | 10 mg | No |
| Example 14 | HSPC | 10 mg | Simvastatin | 10 mg | No |
| Comparative Example 12 | None | --- | Sirolimus | 10 mg | Yes |
| Comparative Example 13 | None | --- | Simvastatin | 10 mg | Yes |

| | | | | | |
|---|---|---|---|---|---|
| * Sirolimus made by Sigma Corp. Simvastatin made by Sigma Corp. | | | | | |

The present application is based on Japanese Patent Application No. 2009-196679 filed on August 27, 2009, the entire content of which is incorporated by reference herein.

### Description of Reference Numerals

- 1: Balloon catheter
- 2: Depth marker
- 3, 6: Guide wire
- 4: Guide wire opening
- 5: Contrast marker
- 7: Hub
- 8: Proximal shaft
- 9: Intermediate part
- 10: Distal shaft
- 11: Inner shaft
- 12: Reinforcing member
- 13: Distal tip
- 21: Balloon
- 22: Drug releasing layer
- 23: Balloon film
- 24: Lumen

## Claims

1. A medical device for drug delivery comprising a base material and a drug releasing layer, the drug releasing layer comprising a drug and a phospholipid, wherein least a part of a surface of the base material is coated with the drug releasing layer, herein the drug and the phospholipid form a solid dispersion.

2. The medical for drug delivery as defined in Claim 1, wherein the solid dispersion is a solid solution.

3. The medical device for drug delivery as defined in Claim 1. wherein the phospholipid is a monomolecular compound.

4. The medical device for drug delivery as defined in Claim 1, wherein the phospholipid is at least one selected from the group consisting of phosphatidylcholine, phosphatidylglycerol, phosphatidic acid, phosphatidylethanolamine, phosphatidylserine, phosphatidylinositol, phosphatidylinositol polyphosphoric acid, sphingomyelin, cardiolipin, partially hydrogenated product thereof, and completely hydrogenated product thereof.

5. The medical device for drug delivery as defined in Claim 1, wherein the phospholipid is phosphatidylcholine or a completely hydrogenated product thereof.

6. The medical device for drug delivery as defined in Claim 1, wherein the drug is insoluble or substantially insoluble in water.

7. The medical device for drug delivery as defined in Claim 1, wherein the drug is at least one selected from the group consisting of an anticancer drug, immunosuppressive drug, antibiotic, antirheumatic drug, antithrombotic drug, HMG-CoAreductase inhibitor, insulin resistance improver, ACE inhibitor, calcium antagonist, antihyperlipidemic drug, integrin inhibitor, antiallergic drug, antioxidant, GP IIb/IIIa antagonist, retinoid, flavonoid, carotenoid, lipid improver, DNA synthesis inhibitor, tyrosine kinase inhibitor, antiplatelet drug, antiinflammatory drug, tissue-derived biomaterial, interferon, and nitrogen monoxide generation promoting substance.

8. The medical device for drug delivery as defined in Claim 1, wherein the drug is paclitaxel.

9. The medical device for drug delivery as defined in Claim 1, wherein the medical device is suitable for contact with a lumen wall tissue of a living body, and the medical device is removable from the living body.

10. The medical device for drug delivery as defined in Claim 1, wherein the base material is a balloon catheter.

11. A method for producing a medical device for drug delivery, the method comprising:
applying a solution of a drug and a phospholipid dissolved in an organic solvent, to a base material, and
subsequently removing said solvent by evaporation to for a drug releasing layer on at least a part of a surface of the base material.
